# EUROPEAN PATENT APPLICATION

(11) **EP 1 674 080 A1**
(43) Date of publication of application: **28.06.2006**
(21) Application number: 04030782.9
(22) Date of filing: 24.12.2004
(51) Int. Cl.: A61K 9/14

(54) **Solid pharmaceutical composition comprising valsartan**

(71) Applicant: KRKA, D.D., Novo Mesto, 8501 Novo mesto (SI)
(72) Inventor: Vrbinc, Miha, 8000 Novo mesto (SI); Slanc, Janika, 1270 Litija (SI); Rangus, Marija, 8310 Sentjernej (SI); Zupancic, Silvo, 8000 Novo mesto (SI); Bevec, Franci, 8210 Trebnje (SI)
(74) Representative: HOFFMANN EITLE

(57) **Abstract**

This invention relates to a solid pharmaceutical composition containing valsartan particles characterized in that the D₅₀ of said valsartan particles is 150 µm or below and that the valsartan particles have a maximum diameter of no more than 1100 µm, as determined by electron microscopy.

## Description

### Field of the Invention

The invention relates to a solid pharmaceutical composition comprising valsartan and a process for its preparation.

Valsartan of formula (I) is chemically described as *N*-(1-oxopentyl)-*N*-{4- [2-(1H-tetrazole-5-yl)phenyl ]-benzyl} -L-valine. Because of its ability to inhibit the angiotensin-converting enzyme, it is widely used for the treatment of hypertension and related diseases and conditions. As an angiotensin II receptor antagonist, valsartan avoids the side-effects of calcium antagonists and shows high stability and obvious curative effects.

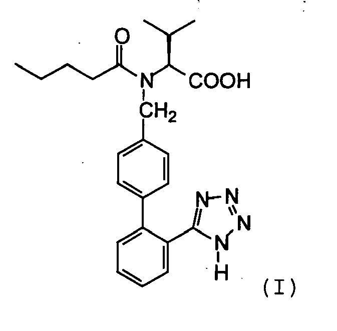

In the context of this invention, the term »valsartan« also includes pharmaceutically acceptable salts, hydrates and solvates of a compound having formula (I).

### Background of the Invention

Certain solid pharmaceutical compositions comprising valsartan and processes for their preparation are known.

Thus, EP-A-0443983 describes a pharmaceutical composition of valsartan and a process for its preparation. Valsartan, lactose and maize starch are mixed and granulated with an ethanolic dispersion of gelatine. After drying, the rest of maize starch, talc, magnesium stearate and colloidal silica (highly dispersed) are admixed to the granulate and compressed into tablets. Tablets can be film-coated.

In another embodiment of this application, valsartan, lactose and maize starch are mixed and granulated with a dispersion of maize starch in warmed water. After drying, the rest of maize starch, talc and calcium stearate are admixed to the granulate and compressed in cores. Cores are coated with a dispersion of hydroxypropylmethylcellulose and shellac in dichloromethane.

EP 0914119 and EP 1410797 disclose a technological procedure of making oral dosage forms of valsartan which comprises the following steps:
- grinding the active agent (e.g. valsartan) and pharmaceutically acceptable additives,
- subjecting a mixture of the ground active agent and additives to compression to form a comprimate (the compacted mass),
- converting the comprimate to form a granulate and
- compressing the granulate to form the solid oral dosage form.
The process is carried out in the absence of water, i.e. it is a dry granulation/compression method.

WO 00/38676 further describes solid oral dosage forms comprising valsartan (20 to 65%), microcrystalline cellulose (31 to 65%) and crospovidone (2 to 13%). Further, a solid oral dosage form comprising valsartan and microcrystalline cellulose in a weight ratio from 2.5 :1 to 0.3:1 is claimed. The method for preparing such compositions is again dry granulation, i.e. works in the absence of water.

WO 01/97805 claims a composition of valsartan or a pharmaceutically acceptable salt or hydrate thereof and a disintegrant in a weight ratio of between 5.1:1 and 0.5:1. The suggested method for preparing such compositions is again dry granulation, i.e. works in the absence of water.

Thus, while one early prior art document (EP-A-0443983) discloses the ethanol-based or water-based granulation and the organic coating of a pharmaceutical composition comprising valsartan, the great majority of the relevant prior art rather suggests that the presence of water in the formulation and coating of valsartan should be avoided. This can be taken from the fact that later inventions trying to provide improved formulations over the one disclosed in EP-A-0443983 mainly focussed on dry granulation/compression methods and organic solvent-based coating, i.e. require the absence of water in the technological process of tablet preparation. Problems that can arise from the use of water in any step of the tablet preparation process are losses in process efficiency, stability, bioavailability, etc. However, the present invention overcomes those problems arising from aqueous granulation and aqueous coating procedures.

Ethanol used in wet granulation is problematic from safety reasons and should be limited by GMP and other quality-based requirements. For example, special equipment (explosion-resistant apparatuses) used in the stages where ethanol is used as a solvent must be available. Secondly, ethanol has to be recycled for environmental protection and safety reasons. A process where ethanol is used is not the first choice also from an economical point of view. All precautions that should be undertaken make a process cost-ineffective and expensive.

Gelatine, when used in an ethanolic dispersion for a granulating process, can be problematic because of its natural source from animals and its potential risk of BSE (bovine spongiform encephalopathy or mad cow disease) exposure. Gelatine is a term for a mixture of purified protein fractions obtained either by partial acidic hydrolysis or by partial alkaline hydrolysis of animal collagen. Moreover, gelatine stems from natural sources, so that it is hard to assure the constance of the product and the properties thereof.

Moreover, ethanolic granulation as described in the prior art makes it difficult to repeat the procedure in other types of equipment for granulation, e.g. in a high-shear granulator or fluid-bed granulator. The wetted mass (after addition of the granulation dispersion to a mixture of valsartan and excipients) is hardened and impossible to dry in conventional drying equipment. We presume that a part of the valsartan is dissolved by ethanol which results in the formation of very strong physical forces between valsartan and excipients that are granulated together. Binders selected from the group of starches (e.g. maize starch or potato starch) have a relatively high water content (up to 15%) and are not suitable for use in wet water granulation because of their multiple function: beside binding properties, they are also disintegrants, diluents and glidants; their role cannot be defined in only one way. Talc as glidant and lubricant was found to be problematic in a wet aqueous granulation process due to the bad flowability of the compression mixture.

Dichloromethane is in the class of solvents that are of limited use in the pharmaceutical industry due to the problem of residual solvents and due to its inherent toxicity. For the validation of the residual solvent detection method, its precision should be determined. Dichloromethane also has to be recycled for environmental protection and safety reasons. Moreover, a process where dichloromethane is used is not the first choice from an economical point of view either. All precautions that should be undertaken render a process cost-ineffective and expensive. Shellac, like gelatine, is a naturally occuring material and it is therefore hard to assure that it has constant properties.

In general, the formulation of valsartan for effective oral administration to a subject has hitherto been complicated by the unique physical and chemical properties of the compound, particularly its low solubility in aqueous media.

In particular, a need exists for effective orally deliverable valsartan formulations possessing one or more of the following:
- appropriate particle size distribution;
- appropriate solubility;
- appropriate disintegration time;
- appropriate dissolution profile;
- appropriate processability;
- appropriate economics of the excipients;
- appropriate economics of the preparation process.

### Summary of the Invention

In a first aspect, the present invention relates to a solid pharmaceutical composition containing valsartan particles characterized in that the D₅₀ of said valsartan particles is 150 µm or below, preferably 130 µm or below and more preferably 110 µm or below. Ideally, at least 20% of the valsartan particles of said composition have a diameter in the range of 0.02 to 50 µm and/or at least 35% of the valsartan particles have a diameter in the range of 0.02 to 100 µm. In the final composition, the valsartan particles have a maximum diameter of 1100 µm, as determined by electron microscopy. More preferably, the valsartan particles have a maximum diameter of 1000, 950 or (most preferably) 900 µm.

In a further aspect, the present invention relates to a solid pharmaceutical composition comprising 30-70 wt.-% of valsartan, 10-70 wt.-% of a diluent, 1-20 wt.-% of a disintegrant, 1-20 wt.-% of a binder and 1-10% of a lubricant, preferably 40-60 wt.-% of valsartan, 30-60 wt.-% of a diluent, 1-15 wt.-% of a disintegrant, 1-15 wt.-% of a binder and 1-8 wt.-% of a lubricant, and most preferably 45-55 wt.-% of valsartan, 35-50 wt.-% of a diluent, 1-10 wt.-% of a disintegrant, 1-10 wt.-% of a binder and 1-6 wt.-% of a lubricant. This solid pharmaceutical composition preferably contains valsartan particles of a particle size as defined above. It may contain further additives other than the ones mentioned above, but in a preferred embodiment the solid pharmaceutical composition according to the invention consists only of 30-70 wt.-% of valsartan, 10-70 wt.-% of a diluent, 1-20 wt.-% of a disintegrant, 1-20 wt.-% of a binder and 1-10% of a lubricant (preferred ranges are the same as indicated above).

In yet a further aspect, the present invention relates to a solid pharmaceutical composition comprising the above-indicated components wherein the weight ratio of valsartan to disintegrant is from 20:1 to 7:1, the weight ratio of valsartan to binder is from 50:1 to 5:1, the weight ratio of valsartan to lubricant is from 30:1 to 5:1, the weight ratio of disintegrant to binder is from 5:1 to 0.5:1, the weight ratio of disintegrant to lubricant is from 5:1 to 0.5:1 and the weight ratio of binder to lubricant is from 5:1 to 0.5:1. Again, this pharmaceutical composition preferably contains valsartan particles of a particle size as defined above.

The solid pharmaceutical composition of the present invention is preferably a tablet which may or may not be coated. In a further preferred aspect, at least 75 wt.-% of the pharmaceutical composition of the present invention are dissolved in a standard acetate buffer of pH=4.5 in 30 minutes.

Moreover, the present invention relates to a process for preparing a pharmaceutical composition as defined in any of the above aspects which comprises the following steps:
- providing valsartan particles having a maximum diameter of 1100 µm,
- granulating a mixture of excipients using water or an aqueous dispersion as granulation liquid to obtain a granulate,
- adding valsartan and further excipients to said granulate to give a compression mixture,
- compressing the compression mixture to the desired form, and
- optionally, applying a coating.

In a further variant, the present invention relates to a process for preparing a pharmaceutical composition as defined in any of the above aspects which comprises the following steps:
- providing valsartan particles having a maximum diameter of 1100 µm,
- granulating a mixture of valsartan and excipients using water or an aqueous dispersion as granulation liquid to obtain a granulate,
- adding further excipients to said granulate to give a compression mixture,
- compressing the compression mixture to the desired form, and
- optionally, applying a coating.

Further preferred embodiments are described in the appended dependent claims.

### Detailed Description

### Particle Diameter

The solid pharmaceutical compositions of the present invention contain valsartan in particulate form. The particle size of valsartan is believed to be an important parameter of the formulation affecting the clinical effectiveness due to its solubility characteristics. In the present invention, the diameter of the valsartan particles is therefore within defined specifications. Moreover, the particle distribution is more homogeneous than in the prior art.

The term "particle size" as used herein refers to the volume diameter of valsartan particles, as determined by laser light scattering using a Malvern-Mastersizer Apparatus MS 2000. D₅₀ means that 50% of particles (V/V) have a higher volume diameter than the indicated value. The term "valsartan particle" means a particle that contains valsartan or, preferably, a particle that essentially or completely consists of valsartan.

In the present invention the pharmaceutical compositions of valsartan have a valsartan particle size such that D₅₀ is below 150 µm, preferably below 130 µm, more preferably below 110 µm, even more preferably below 90 µm and most preferably below 85 µm.

More than 20%, preferably more than 30% of the valsartan particles should preferably be in range of 0.02-50 µm and more than 35%, preferably more than 45% of the valsartan particles should be in the range of 0.02-100 µm.

The particles to be subjected to the particle size measurement are first suspended in vegetable oil (i.e. sunflower oil) and then subjected to a size determination in a Malvern Mastersizer MS 2000 instrument. Usually, 100-800 mg of substance are dispersed in 5-8 ml of vegetable oil. No solubilizers or surfactants are used.

According to manufacturer's information, the Malvern Mastersizers allow for a measurement of particle size distributions in the range of 20 nm to 2000 µm, with a precision of better than 0.5%. Particle sizes are determined by measuring the angular distribution of laser light scattered by a homogeneous suspension of particles. The size distribution is determined from the light scattering data using the theory of light scattering developed by Gustav Mie.

The specified particle size of the valsartan particles according to the invention results in a specific surface area of 1 to 5 m²/g, preferably 2 to 4 m²/g. The specific surface area is defined by the BET method.

In the pharmaceutical composition according to the present invention the valsartan particles have a maximum diameter of 1100 µm, preferably 1000 µm, more preferably 950 µm and most preferably 900 µm. This (maximum) particle diameter is determined as a 2-dimensional diameter by electron microscopy.

Should the valsartan to be used for the preparation of a pharmaceutical composition according to the present invention comprise or consist of larger particles, it is milled (and/or crunched) using pharmaceutically approved equipment for reducing particle sizes, e.g. a Fitz-Patrick Homoloid® mill Model JT or a Frewitt sieving machine before the granulation and compression steps. When a mesh is used as a part of the equipment used, it should have openings of a defined size, e.g. openings of a round shape with diameter of 0.1 mm - 6.5 mm, preferably 0.1 mm - 3 mm, most preferably 0.1 - 1 mm. The variation of the opening size should not be more than 0.05 mm. The conditions of the initial milling process are normal room temperature (22±3°C) and normal pressure (1000±50 mbar). The substance is milled/crunched in the solid state without the aid of a dispersion medium or the like.

A reduction of the particle size generally tends to improve the bioavailability of valsartan. A decrease in the valsartan particle size compensates the poor solubility properties of valsartan from granulate which is obtained by the compression of a mixture of valsartan and excipients to form a comprimate, followed by converting the comprimate to a granulate as it is described in prior art. Adjusting the particle size of valsartan in the range of the present invention will reduce the time needed for the preparation process and represents a significant economical advance in the manufacturing of effective formulations.

Experiments conducted by the inventors have shown that disintegration of tablets prepared by dry granulation does not lead to fast dissolution because the tablets disintegrate into granules and valsartan must dissolve from these granules. Although the formulation of the present invention has a longer disintegration time, smaller particles of valsartan dissolve at the same time as the tablet disintegrates.

The disintegration test can be performed in the same media as the dissolution profile test described below, e.g. at pH=4.5 in acetate buffer, or in 0.1 M HCl or in purified water. Purified water at 37°C is often used as a reference disintegration medium. A disintegration test on cores is performed as in-process control on an Erweka apparatus in purified water at 37°C as described in the last edition of Ph. Eur.

### Components of the pharmaceutical composition

The solid pharmaceutical compositions of the present invention consist of the active ingredient valsartan and excipients. The compositions can be in the form of tablets, pills, powders, lozenges, sachets, soft and hard gelatine capsules, suppositories etc. The dosage form is preferably suitable for oral application.

The compositions are preferably formulated in a unit dosage form, each dosage containing about 1 to about 1000 mg, more usually about 40 to about 320 mg of valsartan. The term »unit dosage form« refers to physically discrete units suitable as unitary dosages for human subjects and other mammals, each unit containing a predetermined quantity of valsartan calculated to produce the desired therapeutic effect, in association with a suitable pharmaceutical excipient.

Valsartan can be present in different physical forms, e.g. in an amorphous form, in one or several crystal form(s) (e.g. anhydrous, solvated or hydrated forms), in the form of mixture of different crystal forms (e.g. anhydrous, solvated or hydrated forms) or as a mixture of an amorphous form and crystal form(s) (e.g. anhydrous, solvated or hydrated forms). Each of these forms is included in the term "valsartan" as used in the present invention.

The pharmaceutical excipients particularly include binders, disintegrants, diluents and lubricants. Other and further excipients can also be contained.

### Binders

The composition according to the invention can also comprise binders, such as polyvinylpyrrolidone, microcrystalline cellulose, hydroxyethylcellulose, hydroxypropylcellulose, hydroxypropylmethylcellulose or other cellulose ethers, starch, pregelatinised starch, or polymethacrylate, or mixtures thereof.

It is preferable to use a binder with a good water solubility. In view of the practical insolubility of starch in cold water, there is a strong preference for binders with a very good solubility in cold water. There is a variety of binders that have very good solubility in water, e.g. povidone of different K-values, i.e. low K-values.

When microcrystalline cellulose is used as a binder, its content in the formulation has an influence on the processability of the product if a dry granulation process is used. Higher contents were generally favoured in the prior art. A high content of microcrystalline cellulose has been said to improve processability in wet water granulation, too. However, we managed to avoid a great influence of the amount of microcrystalline cellulose by its partial or complete substitution by lactose and by using a defined particle size of valsartan. It has been surprisingly found that in a wet water granulation process the amount of microcrystalline cellulose can be minimized to less than 30% in weight in formulation and that the weight ratio between valsartan and microcrystalline cellulose can be more than 4:1.

We also managed to prepare tablets in the complete absence of microcrystalline cellulose by substituting this ingredient (partially or completely) with lactose. Lactose is also less expensive than microcrystalline cellulose and is from an economical viewpoint preferred over microcrystalline cellulose.

In a preferred embodiment of the invention the excipients include at least one binder selected from hydroxypropyl cellulose and povidone.

### Disintegrants

Further, the pharmaceutical compositions of the present invention can also contain disintegrants, such as pregelatinsed starch, sodium starch glycolate, microcrystalline cellulose, carboxymethylcellulose sodium (CMC-Na), cross-linked CMC-Na, polacrilin potassium, low-substituted hydroxypropylcellulose or mixtures thereof.

Crospovidone as a water-insoluble tablet disintegrant can be used in up to 5% concentration in direct compression, wet or dry granulation. However, it is preferable to use a disintegrant that can be used in both the wet and dry stages of the granulation process (intra- and extragranulary) so that the wicking and swelling ability of the disintegrant is best utilized. Crospovidone used in the prior art is practically insoluble in water and most common organic solvents. Therefore, in the formulation of valsartan by wet granulation with water, crospovidone is not a disintegrant of first choice.

As a potential disintegrant that can be used intra- and extragranulary, cross-linked carboxymethylcellulose sodium is therefore preferable. Although it is insoluble in water, it rapidly swells to 4-8 times of its original volume on contact with water. It is advantageous to use the disintegrant in an amount where the weight ratio between valsartan and disintegrant is from 7:1 to 20:1, preferably around 10:1.

It is particularly preferred that the excipients include at least one disintegrant selected from starch and carboxymethylcellulose sodium, e.g. a cross-linked carboxymethylcellulose sodium.

### Diluents

The pharmaceutical compositions according to the invention can further contain diluents such as microcrystalline cellulose, powdered cellulose, lactose (anhydrous or monohydrate), compressible sugar, fructose, dextranes, other sugars such as mannitol, sorbitol, lactitol, saccharose or a mixture thereof, siliconised microcrystalline cellulose, calcium hydrogen phosphate, calcium carbonate, calcium lactate or mixtures thereof.

Preferably, the excipients include at least one diluent selected from microcrystalline cellulose (however, this diluent should only be used in an amount of less than 30 weight %) and lactose monohydrate.

### Lubricants

The composition according to the invention can also comprise lubricants, such as stearic acid, magnesium stearate, calcium stearate, sodium lauryl sulphate, hydrogenated vegetable oil, hydrogenated castor oil, sodium stearyl fumarate, macrogols, or mixtures thereof.

It is preferred that the excipients include at least one lubricant, selected from stearic acid, magnesium stearate, calcium stearate and sodium lauryl sulphate, more preferably from stearic acid, magnesium stearate and calcium stearate.

### Preferred quantities of ingredients

The preferred solid pharmaceutical composition of the present invention comprise (all percentages are weight-based):
- 30-70%, preferably 40-60%, more preferably 45-55% of valsartan,
- 10-70%, preferably 30-60%, more preferably 35-50% of diluent,
- 1-20%, preferably 1-15%, more preferably 1-10% of disintegrant,
- 1-20%, preferably 1-15%, more preferably 1-10% of binder,
- 1-10%, preferably 1-8%, more preferably 1-6% of lubricant.

It is advantageous to define the exact ratio between valsartan, binder and lubricant due to the great influence of the valsartan particle size on the processability, dissolution characteristics and bioavailability of the formulation.

It was surprisingly found that the quantity of some excipients can affect the bioavailability of valsartan. The lubricant quantity is one of the most crucial factors that have an impact on both the compression step of the formulation process (i.e. it prevents sticking on the punches) and the bioavailability of valsartan. An increase of the quantity of e.g. magnesium stearate (a hydrophobic lubricant) in the formulation retards drug dissolution and causes slower drug absorption in the body. Thus, the bioavailability of a valsartan-containing pharmaceutical composition according to the present invention can be controlled by adding a suitable amount of lubricant, most preferably magnesium stearate.

Although the lubricant is *per definitionem* an excipient, i.e. an inactive component of the formulation, it may influence the bioavailability of valsartan as was found out in our formulation experiments. This influence is more pronounced in formulations like the ones of the present invention where valsartan has a smaller particle size and, therefore, a larger specific surface. In this case, magnesium stearate covers the valsartan particles more effectively and therefore makes valsartan particles more hydrophobic due to its own hydrophobic characteristics which results in a certain decrease in bioavailability of valsartan.

Thus, the present invention uses an appropriate amount of lubricant (most preferably magnesium stearate) in combination with the particle size of valsartan to achieve the desired bioavailability. While a smaller particle size would in principle allow for the use of larger amounts of a lubricant such as magnesium stearate without sacrificing bioavailability, an upper limit for the amount of lubricant results from process technological aspects. For example, if the amount of magnesium stearate is too high, the hardness of the tablets would be reduced so that film-coating would no longer be possible. Excessive amounts of lubricant may also reduce the dissolution profiles and as a consequence result in a too low bioavailability.

### Coatings

Optionally, cores/tablets can be coated with conventional materials used for film coating, i.e. as described in Pharmaceutical Coating Technology, 1995, edited by Graham Cole. Film coating formulations usually contain the following components: polymer(s), plasticizer(s), colourant(s)/opacifier(s), vehicle(s). In film coating suspension the minor quantities of flavours, surfactants and waxes can be used. The majority of the polymers used in film coating are either cellulose derivatives, such as the cellulose ethers, or acrylic polymers and copolymers.

Occasionally encountered are high molecular weight polyethylene glycols, polyvinyl pyrrolidone, polyvinyl alcohol and waxy materials.

Typical cellulose ethers are hydroxyethylcellulose, hydroxypropylcellulose, hydroxypropylmethylcellulose and methylcellulose. Acrylic polymers comprise a group of synthetic polymers with diverse functionalities. Some of them can be further modified to enhance swelling and permeability by the incorporation of materials such as water soluble cellulose ethers and starches in order to ensure complete disintegration/dissolution of the film.

The commonly used plasticizers can be categorized into three groups: polyols (glycerol, propylene glycol, macrogols), organic esters (phthalate esters, dibutyl sebacetate, citrate esters, triacetin), oils/glycerides (castor oil, acetylated monoglycerides, fractionated coconut oil).

Colourants/opacifiers are classified into several groups: organic dyes and their lakes, inorganic colours, natural colours. Combination of different materials form each group can be combined in defined ratios. Film coating suspensions can be used as ready-to-make preparations which are available on the market.

Film coating dispersion can be prepared by using different solvents (water, alcohols, ketones, esters, chlorinated hydrocarbons), preferably water.

A composition of coating suspension (calculated on dry material) is particularly preferred which comprises:
- 1-99% by weight of polymer, preferably 1-95% of polymer,
- 1-50% by weight of plasticizer, preferably 1-40% of plasticizer,
- 0.1-20% of colourant/opacifier, preferably 0.1-10% of colourant/opacifier.

### Preparation Process

The present pharmaceutical formulations are prepared by known technological procedures, e.g. compression or wet aqueous granulation, using well known and readily available excipients. In the preparation of the compositions of valsartan, the active principle will usually be mixed with an excipient or mixture of excipients, or diluted by an excipient or mixture of excipients, or enclosed within an excipient or mixture of excipients which may be in the form of a capsule, sachet, paper or other container. When the excipient serves as a diluent, it may be a solid, semisolid or liquid material which acts as a vehicle or medium for the valsartan.

However, we surprisingly found out that the inventive solid oral dosage form which is prepared by wet water granulation can be stable at ICH stability testing conditions. Further, the absence of intra-process compression of valsartan with additives and converting the mixture to a granulate shortens the preparation process in an economical way. Nevertheless, drying a granulate, prepared in a wet aqueous granulation process, in a fluid-bed dryer enables the preparation of round-shaped particles of granulate consisting of valsartan and excipient(s), which provides a reproducible and processable formulation. This can otherwise be a problem due the high weight % of valsartan (>10%) in the solid oral dosage form.

The wetting of a mixture of valsartan and excipient(s) can be performed in conventional granulation equipment by spraying of water or an aqueous granulating liquid onto an excipient or mixture of excipients by conventional pharmaceutical techniques. Wetting can also be effected by direct addition of water or an aqueous granulating liquid to a mixture of excipients during a mixing operation in a proper mixing device, e.g.a high-shear mixer. The term "aqueous granulating liquid" refers to an aqueous dispersion which contains purified or demineralised water as a diluent and a solid substance which is dispersed, suspended or dissolved in the diluent. The dispersed substance can have known functions of excipients, the function of a suspending agent or binding agent, preferably binding agent.

The mixing of excipients or of excipients with valsartan may be effected in conventional devices used for mixing of powders, e.g. motionless (passive) mixers, fluidized bed, diffusion, biconic diffusion, biconic, turbular, cubic, planetary, Y-, V-shaped or high-shear mixers.

For the granulation, conventional drying devices such as a fluid-bed dryer or drying chambers can be used.

In the processes according to the invention as described above the compression, in particular to tablets, can be effected using an automatic rotary compressing machine from different manufacturers of equipment for use in pharmaceutical industry.

Conventional equipment can be used for applying a film coating, such as a Wurster coating system or conventional coating pans for use in pharmaceutical industry.

The process for preparing the pharmaceutical composition according to the invention can be carried out as a granulation process or a direct compression process. In each case, valsartan is first prepared according to a suitable synthetic process and then purified, e.g. by crystallization (see examples) or other means. Then, the size of the valsartan particles is determined and if it is found that there are particles having a diameter of more than 1100 µm (or in preferred embodiments more than e.g. 1000, 950, or 900 µm), then this valsartan will be milled or crunched to a smaller size.

In a preferred embodiment, the wet aqeuous granulation process comprises:
- providing valsartan having a maximum particle diameter of 1100 µm, and, in addition, preferably a D50 of less than 150 µm.
- granulating a mixture of excipients using water or a water-based dispersion as granulation liquid to obtain a granulate,
- addition of said valsartan particles and excipients to the granulate to give a compression mixture,
- compressing the compression mixture to the desired form, and
- optionally, applying a coating.
   In this embodiment, a granulate is prepared without including valsartan thereto.
   In another preferred embodiment, the wet water granulation process comprises
- providing valsartan having a maximum particle diameter of 1100 µm, and, in addition, preferably a D50 of less than 150 µm.
- granulating a mixture of said valsartan particles and excipients using water or a water-based dispersion as granulation liquid to give a granulate,
- addition of excipients to the granulate to give a compression mixture,
- compressing the compression mixture to the desired form, and
- optionally, applying a coating.
   Thus, in this embodiment a granulate is prepared which includes valsartan.
   Further, a preferred embodiment of a direct compression process comprises
- providing valsartan having a maximum particle diameter of 1100 µm, and, in addition, preferably a D50 of less than 150 µm.
- mixing valsartan and excipients to give a compression mixture,
- compressing the compression mixture to the desired form, and
- optionally, applying a coating.

It has been surprisingly found out that the particle size is connected to the main pressure in the compressing process. A smaller particle size of valsartan results in that a lower main pressure at compression process is needed. Furthermore, the hardness of cores or tablets is dependent on setting up the parameters of compression, e.g. main pressure. It is preferred that a processable formulation is produced at maximum compression speed so that the production time is shortened to a minimum, while the parameter settings do not change significantly during the process and remain the parameters of the product within the prescribed specification. It has been found out that a low main pressure (below 25 kN, preferably below 20 kN, more preferably below 15 kN) is sufficient to produce cores or tablets with a hardness that is sufficient to perform e.g. film coating and/or packaging of final product in primary packaging, i.e. contact packaging without damaging the solid oral dosage form. The hardness of cores or tablets depends on the amount of valsartan incorporated:
- 40 mg of valsartan should be incorporated with a hardness not lower than 15 N for both round and oblong core or tablet shape;
- 80 mg of valsartan should be incorporated with a hardness not lower than 30 N for both round and oblong core or tablet shape;
- 160 mg of valsartan should be incorporated with a hardness not lower than 45 N for both round and oblong core or tablet shape;
- 320 mg of valsartan should be incorporated with a hardness not lower than 60 N for both round and oblong core or tablet shape.

An influence of the manufacturing process on the disintegration rate of cores or tablets has been found. Tablets produced by dry granulation rapidly disintegrate in water into large particles, i.e. granules. However, this phenomenon does not result in extremely rapid dissolution as a consequence of fast disintegration. A disintegration test, which is performed as an in-process control in the compression process, is nevertheless a good predictor of the dissolution rate of tablets.

Hardness and disintegration are strongly connected in formulations of valsartan. There is a wish to select and specify a hardness of cores that is suitable for a film coating process. Too soft cores result in the crushing of cores and tablets and fail to meet the specification of the final product. The range between minimum, optimum and maximum hardness has to be determined very precisely because the disintegration of cores is connected to the hardness. Disintegration further influences the dissolution characteristics as described above.

Valsartan is a poorly soluble substance with poor permeability. Therefore, a dissolution test is one of the most crucial factors that can be chosen as a »method-of-choice« for selection of a formulation that is suitable for use in bioavailability studies because it is of great value to use a suitable analytical tool for selection of a formulation for use in bioavailability studies.

In the dissolution of a pooly soluble substance such as valsartan, the particle size distribution is very important. Small particles used in the formulation result in faster dissolution in selective dissolution media. On the contrary, larger particles of the active ingredient result in slower dissolution in selective dissolution media as has been proven in our work. There is a need to make a compromise in the particle size distribution that takes each of processability, e.g., in the compression process, analytics, e.g., the dissolution rate, and pharmacokinetics, e.g. bioavailability, into account.

The exact ratio between valsartan of defined particle size parameters, e.g. particle size distribution, diluent and disintegrant is preferably optimized.

The influence of the valsartan particle size was found to have a strong influence on the compression (tabletting) process. Although there is no significant difference between wet aqueous granulation of valsartan of different particle size distribution, i.e. larger and smaller particles, and excipients, they do have an influence on the compressibility of the compression mixture. Larger particles of valsartan in a formulation with microcrystalline cellulose in an amount of less than 30% by weight in the formulation or the complete substitution of microcrystalline cellulose by lactose result in a high main pressure that must be used on tabletting machine for compression. In contrast, smaller particles incorporated in the same composition result in a very compressible material that can be compressed using a low main pressure on the tabletting machine.

It has been surprisingly found that the shape of the tablet (e.g. round and oval) does not influence the crucial parameters of compression, i.e. the main pressure. The selection of size of both round and oval tablets depends on both the physiologically active amount of valsartan and the weight of the final product, e.g. tablet.

A technical problem in the valsartan administration is to find an economically improved valsartan solid oral dosage form formulation that provides safe and effective oral administration to a subject. Wet aqueous granulation and water-based film coatings were found to solve different problems arising from facts described in prior art. Water has unexpectedly no significant influence on the stability of the product. Hence, it improves the processability of a formulation of defined composition according to the invention where valsartan of a specified particle size distribution and/or a valsartan formulation of a specific composition is used.

It was surprisingly found that the inventive composition of the solid oral dosage form that is prepared by direct compression or wet aqueous granulation can be stable at ICH stability testing conditions. Further, the absence of intra-process compression of valsartan with additives and conversion of the mixture to a granulate, as it is done in dry granulation, shortens the technological process in an economically favourable way. Nevertheless, drying the granulate as prepared in a water granulation process, in e.g. a fluid-bed dryer enables the preparation of round-shaped particles of a granulate consisting of valsartan, i.e. valsartan that allows for a reproducible and processable formulation. This can be otherwise a problem due the high weight % of valsartan (>10%) in the solid oral dosage formulation. It has been found that in the compression process, a very low main pressure can be used (below 15 kN) in an automatic compressing machine, e.g. of Kilian type, independent of size and shape of punches.

### Dissolution Profile

The solid pharmaceutical compositions of the present invention preferably have a dissolution profile of not less than 75 wt.-% in an acetate buffer of pH=4.5 in 30 minutes.

The dissolution test is performed using a paddle apparatus according to Ph. Eur or an apparatus 2 according to USP. It is performed on at least 3 solid pharmaceutical compositions, e.g. tablets, at a temperature of the dissolution medium of 37±0.5°C. The speed of rotation of the paddle is 50 rpm. The volume of the dissolution medium used is 900 ml. The percentage of dissolved valsartan can be measured by UV spectrophotometry.

The following examples illustrate the invention and are not intended to restrict the scope of the invention in any way. In the following examples, the water content was determined as loss on drying on a Mettler Toledo HR73 halogen moisture analyzer. The particle size was determined in a Malvern Mastersizer MS 2000, using a suspension of valsartan in vegetable oil (sunflower oil of Oljarica Kranj).

### Example 1

240 g of valsartan, 72 g of lactose monohydrate, 63 mg of sucrose and 66 g of microcrystalline cellulose were homogenised in a fluid-bed mixer/granulator at an inlet air temperature of 53.2°C until the temperature of the mixture was 40.7°C for 4 minutes.

12 g of povidone were dispersed in 108 g of purified water and sprayed onto the mixture of valsartan and excipients as listed above at an inlet air temperature of 53.2°C-54.1°C for 3 minutes. The granulate obtained was dried in a fluid-bed dryer at an inlet air temperature of 54.1°C-58.9°C up to a granulate temperature of 41.2°C for 6 minutes. The water content was 1,66% by weight.

18 g of cross-linked carboxymethylcellulose sodium, 3 g of anhydrous colloidal silica and 6 g of magnesium stearate was added to the granulate obtained above and mixed in a high-shear mixer. The water content of the compression mixture was 1.76% by weight.

The compression mixture was compressed into one side scored cores with a theoretical weight of 320.00 mg. Oval punches have length of 13.5 mm and width 7 mm. Hardness of cores was 90-127 N and disintegration time (purified water, 37°C) 10 minutes.

Cores were coated in an automatic coating pan with water-based film coating suspension, containing hydroxypropylmethylcellulose (69% by weight), titanium dioxide (17%), iron oxide (6.5%) and polyethylene glycol 4000 (7.5%). The theoretical weight of the film coated tablets was 328.00 mg.

### Example 2

240 g of valsartan, 141 g of lactose monohydrate, 60 g of microcrystalline cellulose and 12 g of povidone were homogenised in a high-shear mixer.

120 g of purified water was sprayed onto the mixture of valsartan and excipients as listed above. The granulate obtained was dried in a fluid-bed dryer at an inlet air temperature 52.3°C until the granulate temperature came to 38.0°C for 8 minutes. The water content was 1.72% by weight.

18 g of cross-linked carboxymethylcellulose sodium, 3 g of anhydrous colloidal silica and 6 g of magnesium stearate were added to the granulate obtained above and mixed in a high-shear mixer. The water content of the compression mixture was 1.93% by weight.

The compression mixture was compressed into cores with a theoretical weight of 320.00 mg. The dimension of punches was the same as in Example 1. The hardness of the cores was 123-147 N and the disintegration time (in purified water, 37°C) 8 minutes.

Cores were coated with a film-coating suspension as described in Example 1. The theoretical weight of the film-coated tablets was 328.00 mg.

### Example 3

240 g of valsartan, 141 g of lactose monohydrate, 52.5 g of microcrystalline cellulose and 12 g of povidone were homogenised in a high-shear mixer.

120 g of purified water was sprayed onto the mixture of valsartan and excipients as listed above. The granulate obtained was dried in a fluid-bed dryer at an inlet air temperature of 51.2°C until the granulate temperature reached 38.3°C for 12 minutes. The water content was 1.52% by weight.

18 g of cross-linked carboxymethylcellulose sodium, 3 g of anhydrous colloidal silica and 13.5 g of magnesium stearate were added to the granulate obtained above and mixed in a high-shear mixer. The water content of compression mixture was 1.56% by weight.

The compression mixture was compressed into cores with a theoretical weight of 320.00 mg. The dimension of punches was the same as in Example 1. Hardness of cores was 96-108 N and the disintegration time (purified water, 37°C) 6 minutes.

Cores were coated with a film-coating suspension as described in Example 1. The theoretical weight of film-coated tablets was 328.00 mg.

### Example 4

240 g of valsartan, 195 g of lactose monohydrate and 12 g of povidone were homogenised in a high-shear mixer.

80 g of purified water were sprayed onto the mixture of valsartan and excipients as listed above. The granulate obtained was dried in a fluid-bed dryer at an inlet air temperature of 51.6°C until the granulate temperature reached 38.1°C for 7 minutes. The water content was 0.90% by weight.

24 g of cross-linked carboxymethylcellulose sodium, 3 g of anhydrous colloidal silica and 6 g of magnesium stearate were added to the granulate obtained above and mixed in a high-shear mixer. The water content of the compression mixture was 0.97% by weight.

The compression mixture was compressed into cores with a theoretical weight of 320.00 mg. The dimension of punches was the same as in Example 1. The hardness of cores was 108-144 N and the disintegration time (purified water, 37°C) 11.5 minutes.

Cores were coated with a film-coating suspension as described in Example 1. The theoretical weight of the film-coated tablets was 328.00 mg.

### Example 5

240 g of valsartan, 204 g of lactose monohydrate and 18 g of povidone were homogenised in a high-shear mixer.

80 g of purified water was sprayed onto the mixture of valsartan and excipients as listed above. The granulate obtained was dried in a fluid-bed dryer at an inlet air temperature of 51.2°C until the granulate temperature reached 37.3°C for 4,5 minutes. The water content was 1.48% by weight.

9 g of cross-linked carboxymethylcellulose sodium, 3 g of anhydrous colloidal silica and 6 g of magnesium stearate were added to the granulate obtained above and mixed in a high-shear mixer. The water content of the compression mixture was 1.55% by weight.

The compression mixture was compressed into cores with a theoretical weight of 320.00 mg. The dimension of punches was the same as in Example 1. The hardness of cores was 100-122 N and the disintegration time (purified water, 37°C) was 13.5 minutes.

The cores were coated with a film-coating suspension as described in Example 1. The theoretical weight of the film-coated tablets was 328.00 mg.

### Example 6

16.0 kg of valsartan (A), 9.0 g of lactose monohydrate, 3.90 kg of microcrystalline cellulose and 400 g of povidone were homogenised in a high-shear mixer. Valsartan (A) had the following particle size distribution (table 1) as determined on a Malvern Mastersizer MS 2000.

**Table 1. Particle size distribution of valsartan (A).**

| Range (µm) | Volume % |
|---|---|
| 0.01-2 | 0.9 |
| 2-5 | 3.3 |
| 5-10 | 5.5 |
| 10-20 | 9.3 |
| 20-50 | 19.1 |
| 50-100 | 20.0 |
| 100-150 | 12.4 |
| 150-800 | 29.4 |

The average particle size (as determined using the Malvern Mastersizer MS 2000 software) was 123 µm.
10% of particles were smaller than 10.3 µm.
10% of particles were larger than 304.3 µm.
50% of particles were larger than 76.6 µm.
38.1% of particles were in the range of 0.02-50 µm and 58.1% in the range of 0.02-100 µm.

12.4 kg of purified water were sprayed onto the mixture of valsartan and excipients as listed above. The granulate obtained was dried in a fluid-bed dryer at an inlet air temperature of 65-71°C until the granulate temperature reached 38.6°C for 70 minutes. The water content was 1.60% by weight.

1.60 kg of cross-linked carboxymethylcellulose sodium, 200 g of anhydrous colloidal silica and 900 g of magnesium stearate were added to the granulate obtained above and mixed in a high-shear mixer. The water content of the compression mixture was 1.80% by weight.

The compression mixture was compressed into cores with a theoretical weight of 320.00 mg. The dimension of punches was the same as in Example 1. The hardness of the cores was 94-120 N and the disintegration time (purified water, 37°C) was 2.5-3 minutes. The main pressure on the compressing machine of Kilian type was 4.9 kN. The cores were coated with a film-coating suspension as described in Example 1. The theoretical weight of the film-coated tablets (A) was 328.00 mg.

In another experiment, the mixture was compressed into one-side scored cores with a theoretical weight of 160.00 mg. Round punches have a diameter of 8 mm and a height of cores of 3.6 to 3.8 mm were used. The hardness of the cores was 52-79 N and the disintegration time (purified water, 37°C) was 3-4 minutes. The main pressure on a compressing machine of the Kilian type was 3.9 kN. Some of the cores were then coated in an automatic coating pan with a water-based film coating suspension, containing hydroxypropylmethylcellulose (75% by weight), titanium dioxide (17%), iron oxide (0.5%) and polyethylene glycol 4000 (7.5%). The theoretical weight of the film-coated tablets was 164.00 mg.

### Example 7

The same composition for 328.00 mg film-coated tablets and the same process was used as in Example 6, but using valsartan (B). The film-coated tablets contained valsartan particles of a size distribution, as determined on a Malvern Mastersizer MS 2000, as shown in the following Table 2.

**Table 2. Particle size distribution of valsartan (B).**

| Range (µm) | Volume % |
|---|---|
| 0.01-2 | 0.7 |
| 2-5 | 1.2 |
| 5-10 | 1.0 |
| 10-20 | 2.6 |
| 20-50 | 9.5 |
| 50-100 | 15.0 |
| 100-150 | 13.1 |
| 150-800 | 56.4 |

The average particle size was 219 µm.
10% of the particles were smaller than 34.7 µm.
10% of the particles were larger than 460.8 µm.
50% of the particles were larger than 179.1 µm.

15.0% of the particles were in the range of 0.02-50 µm and 30.0% were in the range of 0.02-100 µm. The water content in the granulate was 1.78% by weight, while the water content of the compression mixture was 1.80% by weight.

The compression mixture was compressed into cores with a theoretical weight of 320.00 mg. The hardness of cores was 58-82 N and the disintegration time (purified water, 37°C) was 3.5 minutes.

The cores (B) were coated with a film-coating suspension as described in Example 1. The theoretical weight of the film-coated tablets (B) was 328.00 mg.

### Example 8

The same composition for 328.00 mg film-coated tablets and the same process as in Example 6 were used, but using valsartan (C). The film-coated tablets contained valsartan particles of a size distribution, as determined on a Malvern Mastersizer MS 2000, as shown in the following Table 3. Valsartan (C) is valsartan (B) milled in a Fitzpatrick Model JT Homoloid mill.

**Table 3. Particle size distribution of valsartan (C).**

| Range (µm) | Volume % |
|---|---|
| 0.01-2 | 1.4 |
| 2-5 | 4.8 |
| 5-10 | 5.7 |
| 10-20 | 9.4 |
| 20-50 | 25.9 |
| 50-100 | 31.3 |
| 100-150 | 14.1 |
| 150-800 | 7.3 |

The average particle size was 65 µm.
10% of the particles were smaller than 8.2 µm.
10% of the particles were larger than 136.2 µm.
50% of the particles were larger than 53.4 µm.

47.2% of the particles were in the range of 0.02-50 µm and 78.5% were in the range of 0.02-100 µm.

The water content in the granulate was 1.68% by weight, while the water content of the compression mixture was 1.70% by weight. The compression mixture was compressed into cores with a theoretical weight of 320.00 mg at a main pressure on a Kilian compressing machine of 11.5 kN. The hardness of the cores (C) was 118-130 N and the disintegration time (purified water, 37°C) was 10-11 minutes.

At a main pressure on a Kilian compressing machine of 5.8 kN the hardness of cores (C') was 67-92 N and the disintegration time (purified water, 37°C) was 2.5 minutes. Cores (C) and (C') were coated with a film-coating suspension as described in Example 1. The theoretical weight of the film-coated tablets (C) and (C') was 328.00 mg.

The percentages of dissolved valsartan in film-coated tablets, as obtained in Examples 6, 7 and 8 in acetate buffer (pH=4.5) are collected in Table 4.

**Table 4. Percentages of dissolved valsartan in film-coated tablets, as obtained in Examples 6, 7 and 8 in acetate buffer pH=4.5.**

| | % of dissolved valsartan from | | | |
|---|---|---|---|---|
| Time (min) | Film-coated tablets (A) | Film-coated tablets (B) | Film-coated tablets (C) | Film-coated tablets (C') |
| 0 | 0 | 0 | 0 | 0 |
| 5 | 41 | 30 | 13 | 53 |
| 10 | 65 | 48 | 43 | 78 |
| 15 | 77 | 57 | 61 | 87 |
| 20 | 84 | 64 | 72 | 91 |
| 25 | 89 | 70 | 77 | 93 |
| 30 | 92 | 74 | 82 | 95 |
| 35 | n.a. | 78 | 85 | 97 |
| 40 | n.a. | 80 | 88 | 98 |
| 45 | 97 | 83 | 90 | 99 |
| 50 | n.a. | 85 | 91 | 99 |
| 55 | n.a. | 87 | 93 | 100 |
| 60 | 99 | 89 | 94 | 100 |

n.a. = not available

### Example 9

16.0 kg of valsartan, 12.1 kg of lactose monohydrate and 1.2 kg of povidone were homogenised in a high-shear mixer. 9 kg of purified water were sprayed onto the mixture of valsartan and excipients as listed above. The granulate obtained was dried for 30 minutes in a fluid-bed dryer at an inlet air temperature of 64-70°C to a granulate temperature of 35°C. The water content was 1.80% by weight.

1.6 kg of cross-linked carboxymethylcellulose sodium, 200 g of anhydrous colloidal silica and 900 g of magnesium stearate were added to the granulate obtained above and mixed in a high-shear mixer. The water content of the compression mixture was 1.96% by weight.

The compression mixture was compressed into cores with a theoretical weight of 320.00 mg. The hardness of cores was 86-107 N and the disintegration time (purified water, 37°C) 7 minutes. The main pressure on a Kilian compressing machine was 4.5 kN.

Some of the cores obtained above were coated in a automatic coating pan with a water-based film coating suspension, containing hydroxypropylmethylcellulose (69% by weight), titanium dioxide (17%), iron oxide (6.5%) and polyethylene glycol 4000 (7.5%). The theoretical weight of the film-coated tablets was 328.00 mg.

In another experiment the compression mixture was compressed into cores with a theoretical weight of 160.00 mg. The average hardness of the cores was 56-80 N and the disintegration time (in purified water, 37°C) 8-9 minutes. The main pressure on a Kilian compressing machine was 3.3 kN. The cores were coated in an automatic coating pan with a water-based film coating suspension, containing hydroxypropylmethylcellulose (75% by weight), titanium dioxide (17%), iron oxide (0.5%) and polyethylene glycol 4000 (7.5%). The theoretical weight of the film-coated tablets was 164.00 mg.

The percentages of dissolved valsartan in film-coated tablets, as obtained in Example 9, in standard acetate buffer pH=4.5 are collected in Table 5.

**Table 5. % of dissolved valsartan in film-coated tablets, obtained in Example 9 in buffer pH=4.5**

| Time (min) | % of dissolved valsartan |
|---|---|
| 0 | 0 |
| 5 | 15 |
| 10 | 40 |
| 15 | 58 |
| 20 | 68 |
| 25 | 75 |
| 30 | 80 |
| 35 | n.a. |
| 40 | n.a. |
| 45 | 89 |
| 50 | n.a. |
| 55 | n.a. |
| 60 | 93 |

| | |
|---|---|
| n.a. = not available | |

### Example 10

The following preparation examples show how a valsartan can be obtained that has a suitable quality for use in a pharmaceutical composition according to the present invention. At the end of each experiment, the valsartan particle diameter can be checked by electron microscopy and the particles are further crunched or milled if the maximum particle diameter exceeds 1100 µm.
a) Crystallization from isopropyl acetate
   5 g of crude valsartan are dissolved in 50 ml of isopropyl acetate at 50°C. Then the solution is gradually cooled to 20°C in 2h and stirred at this temperature for 18h. Pure valsartan is filtered off and dried under reduced pressure. 3.8 g valsartan are isolated.
b) Crystallization from mixture of isopropyl acetate/cyclohexane (1:1)
   2 g crude valsartan are dissolved in 20 ml of mixture isopropyl acetate/cyclohexane (1:1) at 50°C. Then the solution is gradually cooled to 20°C in 2h and stirred at this temperature for 18h. Pure valsartan is filtered off and dried under reduced pressure. 1.8 g valsartan are isolated.
c) Crystallization from a mixture of isopropyl acetate/heptane (10:1)
   2 g crude valsartan are dissolved in 22 ml of a mixture of isopropyl acetate/heptane (10:1) at 50°C. Then the solution is gradually cooled to 20°C in 2h and stirred at this temperature for 18h. Pure valsartan is filtered off and dried under reduced pressure. 1.5 g valsartan are isolated.
d) Crystallization from methyl acetate
   3 g crude valsartan are dissolved in 18 ml of methyl acetate at 50°C. Then the solution is gradually cooled to 20°C in 2h and stirred at this temperature for 18h. Pure valsartan is filtered off and dried under reduced pressure. 2.6 g valsartan are isolated.

## Claims

1. A solid pharmaceutical composition containing valsartan particles **characterized in that** the D₅₀ of said valsartan particles is 150 µm or below and that the valsartan particles have a maximum diameter of no more than 1100 µm, as determined by electron microscopy.

2. The pharmaceutical composition according to claim 1 **characterized in that** at least 20% of the valsartan particles have a diameter in the range of 0.02 to 50 µm.

3. The pharmaceutical composition according to claim 1 or 2 **characterized in that** at least 35% of the valsartan particles have a diameter in the range of 0.02 to 100 µm.

4. The pharmaceutical composition according to any of the preceding claims **characterized in that** the D₅₀ of said valsartan particles is 120 µm or below.

5. The pharmaceutical composition according to any of the preceding claims **characterized in that** the D₅₀ of said valsartan particles is 90 µm or below.

6. The pharmaceutical composition according to any of the preceding claims wherein the valsartan particles are present in an amorphous form, a crystalline form, as a mixture of different crystalline forms or as a mixture of an amorphous form and one or several crystalline forms.

7. A pharmaceutical composition according to any of the preceding claims comprising 30-70 wt.-% of valsartan, 10-70 wt.-% of a diluent, 1-20 wt.-% of a disintegrant, 1-20 wt.-% of a binder and 1-10% of a lubricant.

8. A solid valsartan-containing pharmaceutical composition comprising 30-70 wt.-% of valsartan, 10-70 wt.-% of a diluent, 1-20 wt.-% of a disintegrant, 1-20 wt.-% of a binder and 1-10% of a lubricant.

9. A pharmaceutical composition according to any of the preceding claims comprising 40-60 wt.-% of valsartan, 30-60 wt.-% of a diluent, 1-15 wt.-% of a disintegrant, 1-15 wt.-% of a binder and 1-8 wt.-% of a lubricant.

10. A pharmaceutical composition according to any of claims 7 to 9 wherein the weight ratio of valsartan to disintegrant is from 20:1 to 7:1, the weight ratio of valsartan to binder is from 50:1 to 5:1, the weight ratio of valsartan to lubricant is from 30:1 to 5:1, the weight ratio of disintegrant to binder is from 5:1 to 0.5:1, the weight ratio of disintegrant to lubricant is from 5:1 to 0.5:1 and the weight ratio of binder to lubricant is from 5:1 to 0.5:1.

11. A pharmaceutical composition according to any of claims 7 to 10 wherein the diluent is selected from microcrystalline cellulose (in which case the amount is less than 30 wt.-%) and lactose monohydrate.

12. A pharmaceutical composition according to any of claims 7 to 10 wherein the disintegrant is selected from starch and crosslinked or uncrosslinked carboxymethylcellulose sodium.

13. A pharmaceutical composition according to any of claims 7 to 10 wherein the binder is selected from hydroxypropylcellulose and povidone.

14. A pharmaceutical composition according to any of claims 7 to 10 wherein the lubricant is selected from stearic acid, magnesium stearate, calcium stearate and sodium lauryl sulphate.

15. A pharmaceutical composition according to any of the preceding claims which is an optionally coated tablet.

16. A pharmaceutical composition according to any of the preceding claims **characterised in that** at least 75 wt.-% of said pharmaceutical composition are dissolved in an acetate buffer of pH=4.5 in 30 minutes.

17. A process for preparing a pharmaceutical composition according to any of the preceding claims which comprises the following steps:
- providing valsartan particles having a maximum diameter of 1100 µm
- granulating a mixture of excipients using water or an aqueous dispersion as granulation liquid to obtain a granulate,
- adding the valsartan particles and further excipients to said granulate to give a compression mixture,
- compressing the compression mixture to the desired form, and
- optionally, applying a coating.

18. A process for preparing a pharmaceutical composition according to any of the preceding claims which comprises the following steps:
- providing valsartan particles having a maximum diameter of 1100 µm
- granulating a mixture of valsartan and excipients using water or an aqueous dispersion as granulation liquid to obtain a granulate,
- adding further excipients to said granulate to give a compression mixture,
- compressing the compression mixture to the desired form, and
- optionally, applying a coating.

19. A process according to any of claims 17 or 18, wherein the compression pressure in the compression step is 25 kN or below.

20. A process according to any of claims 17-19 wherein the valsartan particles have a D₅₀ of 150 µm or below.

21. A process according to claim 20 wherein at least 20% of the valsartan particles have a diameter in the range of 0.02 to 50 µm.
